# EUROPEAN PATENT APPLICATION

(11) **EP 4 702 966 A1**
(43) Date of publication of application: **04.03.2026**
(21) Application number: 24797037.9
(22) Date of filing: 24.04.2024
(51) Int. Cl.: A61K 8/29, A61K 8/81, A61K 8/88, A61Q 1/10

(54) **WATER-BASED LIQUID COSMETIC**

(30) Priority: 28.04.2023 JP 2023074868
(71) Applicant: MITSUBISHI PENCIL COMPANY, LIMITED, Tokyo 140-8537 (JP)
(72) Inventor: INOUE, Kensuke, Fujioka-shi, Gunma 375-8501 (JP)
(74) Representative: Vossius & Partner Patentanwälte Rechtsanwälte mbB
(86) International application number: PCT/JP2024/015999
(87) International publication number: WO 2024/225291

(57) **Abstract**

A water-based liquid cosmetic according to the present invention is housed in a pen-type applicator and contains a titanium oxide pigment, a polyorganic acid or a polyorganic acid salt, emulsion particles containing an acrylates copolymer, and a water-based solvent. The ratio of the mass of the titanium oxide pigment to the mass of the polyorganic acid or the polyorganic acid salt is more than five.

## Description

### FIELD

The present invention relates to a water-based liquid cosmetic.

### BACKGROUND

Conventionally, inorganic pigments have been used as coloring materials in makeup cosmetics such as eyeshadows, eyeliners, eyebrows, and mascaras. Studies have been carried out on issues such as inhibition of aggregation and sedimentation of inorganic pigments in cosmetics and improvement of density of drawn lines.

PTL 1 discloses a water-based liquid cosmetic containing sodium diethylhexyl sulfosuccinate, an inorganic coloring pigment, a water-soluble dispersant, and a film-forming polymer emulsion. In the water-based liquid cosmetic, the content of sodium diethylhexyl sulfosuccinate is 0.01 to 1% by mass based on the total amount of the cosmetic, the content of the inorganic coloring pigment is 3 to 20% by mass, and the inorganic coloring pigment includes carbon black.

PTL 2 discloses a water-based liquid cosmetic containing sodium polyaspartate, at least one pearlescent powder, a water-soluble dispersant, and at least one prescribed metal oxide.

### [CITATION LIST]

### [PATENT LITERATURE]

[PTL 1] Japanese Unexamined Patent Publication (Kokai) No. 2020-70259
[PTL 2] Japanese Unexamined Patent Publication (Kokai) No. 2020-132619

### SUMMARY

### [TECHNICAL PROBLEM]

The present invention provides a water-based liquid cosmetic capable of imparting satisfactory color payoff property even after being left to stand for a prolonged period of time while maintaining satisfactory liquid outflow property, cap-off non-drying property, adhesion, sebum resistance, and water-and-sebum resistance.

### [SOLUTION TO PROBLEM]

The present inventors have carried out intensive research and discovered that the above object can be achieved by the following means, thus completing the present invention. Specifically, the present invention is as follows:
<Aspect 1> A water-based liquid cosmetic stored in a pen-style applicator, wherein
   a titanium oxide pigment, a polyorganic acid or polyorganic acid salt, an emulsion particle comprising an acrylates copolymer, and a water-based solvent are contained, and
   a ratio of mass of the titanium oxide pigment relative to mass of the polyorganic acid or polyorganic acid salt is greater than 5.
<Aspect 2> The water-based liquid cosmetic according to Aspect 1, wherein content of the titanium oxide pigment is 10% by mass or more relative to entirety of the water-based liquid cosmetic.
<Aspect 3> The water-based liquid cosmetic according to Aspect 1 or 2, wherein the polyorganic acid or the polyorganic acid salt is at least one selected from a group consisting of polyaspartic acid, polylactic acid, polyphosphoric acid, and polyacrylic acid or salts thereof.
<Aspect 4> The water-based liquid cosmetic according to any one of Aspects 1 and 3, wherein the emulsion particle comprising the acrylates copolymer is a core-shell emulsion particle.
<Aspect 5> The water-based liquid cosmetic according to any one of Aspects 1 and 4, wherein the water-based solvent contains water and at least one alcohol selected from a group consisting of lower alcohols having 5 or fewer carbon atoms and polyhydric alcohols having 10 or fewer carbon atoms.
<Aspect 6> The water-based liquid cosmetic according to any one of Aspects 1 to 5, further containing a pigment other than the titanium oxide pigment.
<Aspect 7> The water-based liquid cosmetic according to Aspect 6, wherein content of the titanium oxide pigment is 45% by mass or more relative to total amount of the pigment other than the titanium oxide pigment.
<Aspect 8> A pen-style applicator containing the water-based liquid cosmetic according to any one of Aspects 1 to 7.

### [ADVANTAGEOUS EFFECTS OF INVENTION]

According to the present invention, a water-based liquid cosmetic capable of imparting satisfactory color payoff property even after being left to stand for a prolonged period of time while maintaining satisfactory liquid outflow property, cap-off non-drying property, adhesion, sebum resistance, and water-and-sebum resistance can be provided.

### BRIEF DESCRIPTION OF DRAWINGS

[FIG. 1] This drawing is a partial cross-sectional view showing the first aspect of a liquid cosmetic applicator containing the water-based liquid cosmetic of the present invention. The mechanism in FIG. 1 is one example of a rotary delivery type.
[FIG. 2] This drawing is a partial cross-sectional view showing the second aspect of a liquid cosmetic applicator containing the water-based liquid cosmetic of the present invention. The mechanism in FIG. 2 is one example of a cosmetic-integrated applicator type.
[FIG. 3] (a) is a perspective view showing the third aspect of a liquid cosmetic applicator storing the water-based liquid cosmetic of the present invention, and (b) is a partial cross-sectional view of an identical liquid cosmetic applicator to that shown in (a). The mechanism of the liquid cosmetic applicator in (a) and (b) is one example of a collector applicator type.

### DESCRIPTION OF EMBODIMENTS

### <<Water-based liquid cosmetic>>

The water-based liquid cosmetic of the present invention is
a cosmetic stored in a pen-style applicator, wherein
a titanium oxide pigment, a polyorganic acid or polyorganic acid salt, an emulsion particle comprising an acrylates copolymer, and a water-based solvent are contained, and
a ratio of mass of the titanium oxide pigment relative to mass of the polyorganic acid or polyorganic acid salt is greater than 5.

In a conventional water-based liquid cosmetic containing a titanium oxide pigment, the titanium oxide pigment is susceptible to sedimentation and aggregation, and is therefore generally used in a relatively small amount, and if used in a large amount, it is necessary to increase the viscosity to a level not suitable for use as a liquid cosmetic.

The present inventors have discovered that in the water-based liquid cosmetic of the present invention, by containing a small amount of a polyorganic acid or a polyorganic acid salt relative to a titanium oxide pigment, i.e., setting the ratio of the mass of the titanium oxide pigment relative to the mass of the polyorganic acid or polyorganic acid salt to greater than 5, redispersion within an applicator, for example, by an agitating body in the applicator, while maintaining satisfactory liquid outflow property, is facilitated, and thus sedimentation and aggregation of the titanium oxide pigment in the water-based liquid cosmetic (after dispersion) can be inhibited. Accordingly, as a result, a water-based liquid cosmetic suitable for applicators of, for example, eyeliners, having a brush-shaped application portion can be obtained.

The ratio of the mass of the titanium oxide pigment relative to the mass of the polyorganic acid or polyorganic acid salt may be greater than 5.0 and 100.0 or less. It is preferable from the viewpoint of improving the viscosity of the water-based liquid cosmetic that this ratio be greater than 5.0, 5.5 or greater, 6.0 or greater, 6.5 or greater, 7.0 or greater, 7.5 or greater, 8.0 or greater, 8.5 or greater, or 9.0 or greater. It is preferable from the viewpoint of improving the dispersion stability of the titanium oxide pigment that this ratio be 100.0 or less, 90.0 or less, 80.0 or less, 70.0 or less, 60.0 or less, 50.0 or less, 45.0 or less, 40.0 or less, 35.0 or less, 30.0 or less, 25.0 or less, 20.0 or less, 15.0 or less, 14.0 or less, 13.5 or less, or 13.0 or less.

The water-based liquid cosmetic of the present invention may contain an optional nonionic surfactant, or may not contain one. When the water-based liquid cosmetic of the present invention does not contain a nonionic surfactant, color payoff property and cap-off non-drying property tend to be satisfactory.

The water-based liquid cosmetic of the present invention may contain other optional components. As other components, for example, antioxidants, neutralizing agents, ultraviolet absorbers, pH adjusters, moisturizers, cosmetic ingredients, fragrances, viscosity/viscous property modifiers, other dispersants such as polyethylene glycol alkyl ethers, and other coloring materials such as water-soluble dyes and resin particle pigments can be used. The content of the other components may be appropriately determined in ranges where the effect of the present invention is not hindered.

In the water-based liquid cosmetic of the present invention, the viscosity may be 2 mPa·s or more and 15 mPa·s or less. It is preferable from the viewpoint of improving the outflow property of the water-based liquid cosmetic that this viscosity be 15 mPa·s or less, 12 mPa·s or less, 10 mPa·s or less, 9 mPa·s or less, 8 mPa·s or less, or 7 mPa·s or less. This viscosity may be 2 mPa·s or more, 3 mPa·s or more, or 4 mPa·s or more.

The adjustment of the viscosity range above can be carried out by adjusting the average particle size of the pigment used, the type and content of each component, and the dispersion method.

In the present invention, viscosity is measured under the conditions of shear rate of 50 rpm (shear rate: 192 [s⁻¹]) and temperature of 25°C. Measurement of this viscosity can be carried out using, for example, an ELD viscometer (Toki Sangyo).

In the water-based liquid cosmetic of the present invention, from the point of improving solubility and inhibiting skin irritability, it is preferable that the pH, when measured as pH (25°C) using a glass electrode, be in the range of 6 or greater and 9 or less. The adjustment of pH can be carried out using the above pH adjuster.

The water-based liquid cosmetic of the present invention can be prepared by dispersing a titanium oxide pigment, a polyorganic acid or polyorganic acid salt, an emulsion particle comprising an acrylates copolymer, a water-based solvent, and other optional components under known dispersion conditions.

As a dispersion means, for example, a disperser such as a homomixer, a sand mill, an ultrasonic homogenizer, or a high-pressure homogenizer can be used. Particularly, according to these dispersion means, the average particle size of the titanium oxide pigment in the cosmetic (after dispersion) can be adjusted to 300 nm or less. In the present invention, "average particle size" means the value of a histogram-average particle size (D50) calculated on a volume basis in a scattering intensity distribution measured by dynamic light scattering.

For example, when an ultrasonic homogenizer is used as the disperser, a dispersion condition of frequency of 20 to 30 kHz may be adopted. When a high-pressure homogenizer is used as the disperser, a dispersion condition of pressure of 150 to 245 MPa may be adopted. From these dispersion conditions, the average particle size of the titanium oxide pigment in the cosmetic (after dispersion) can be adjusted to 300 nm or less.

The water-based liquid cosmetic of the present invention can be used as a cosmetic, such as for eyeshadow, eyeliner, eyebrow, or mascara.

Hereinafter, each component of the water-based liquid cosmetic of the present invention will be described.

### <Titanium oxide pigment>

As titanium oxide pigments, not only pigments containing titanium oxide as a majority component, but also pigments having surfaces coated with titanium oxide and pigments in which titanium oxide is the core particle and the surface is coated with a resin or a coloring material other than titanium oxide, can be used.

As such titanium oxide pigments, commercially available titanium oxide pigments, titanium black, titanated mica, titanium dioxide-coated mica, titanium dioxide-coated synthetic phlogopite, titanium dioxide-coated bismuth oxychloride, iron oxide-coated titanated mica, Prussian blue-treated titanated mica, carmine-treated titanated mica, and titanium dioxide-coated glass powder can be used. These titanium oxide pigments may be used independently or may be mixed and used.

The content of the titanium oxide pigment may be 1% by mass or more and 25% by mass or less relative to the mass of the water-based liquid cosmetic. It is preferable from the viewpoint of improving color payoff and covering power that this content be 1% by mass or more, 2% by mass or more, 3% by mass or more, 4% by mass or more, 5% by mass or more, 6% by mass or more, 7% by mass or more, 8% by mass or more, 9% by mass or more, 10% by mass or more, 11% by mass or more, 12% by mass or more, 13% by mass or more, 14% by mass or more, 15% by mass or more, 16% by mass or more, or 17% by mass or more. It is preferable from the viewpoint of not excessively increasing the viscosity of the water-based liquid cosmetic and the viewpoint of improving applicability that this content be 25% by mass or less, 24% by mass or less, 23% by mass or less, 22% by mass or less, 21% by mass or less, or 20% by mass or less.

When the water-based liquid cosmetic of the present invention contains a pigment other than the titanium oxide pigment, the content of the titanium oxide pigment may be 5% by mass or more and less than 100% by mass relative to the total mass of the titanium oxide pigment and the pigment other than the titanium oxide pigment. This content may be 5% by mass or more, 10% by mass or more, 15% by mass or more, 20% by mass or more, 25% by mass or more, 30% by mass or more, 35% by mass or more, 40% by mass or more, 45% by mass or more, 48% by mass or more, 50% by mass or more, 55% by mass or more, 60% by mass or more, 65% by mass or more, 70% by mass or more, 75% by mass or more, 80% by mass or more, or 85% by mass or more. This content may be less than 100% by mass, 99% by mass or less, 98% by mass or less, 97% by mass or less, or 96% by mass or less.

The average particle size of the titanium oxide before dispersion may be 100 nm or more and 500 nm or less. This average particle size, for example, may be 100 nm or more, 150 nm or more, 200 nm or more, 220 nm or more, 250 nm or more, or 270 nm or more, and may be 500 nm or less, 450 nm or less, 420 nm or less, 400 nm or less, 370 nm or less, 350 nm or less, or 320 nm or less.

### <Polyorganic acid or polyorganic acid salt>

By using a polyorganic acid and a polyorganic acid salt, the dispersibility of the titanium oxide pigment as a coloring material can be improved.

As the polyorganic acid, for example, at least one selected from the group consisting of polyaspartic acid, polylactic acid, polyphosphoric acid, polyacrylic acid, and polyglutamic acid can be used.

As the polyorganic acid salt, for example, at least one selected from the group consisting of various salts of the above polyorganic acids, specifically polyaspartic acid, polylactic acid, polyphosphoric acid, polyacrylic acid, and polyglutamic acid, can be used. As types of these salts, alkali metal salts, alkaline earth metal salts, ammonium salts, and alkanolamine salts can be used. Among these, sodium salts, potassium salts, and lithium salts are particularly preferable.

Among these polyorganic acids or polyorganic acid salts, particularly, polyaspartic acid or salts thereof can impart satisfactory dispersion stability of the titanium oxide pigment. Sodium polyaspartate is particularly preferable.

The (total) content of these polyorganic acids and polyorganic acid salts may be 0.2% by mass or more and 4.0% by mass or less relative to the mass of the water-based liquid cosmetic. This content may be 0.2% by mass or more, 0.4% by mass or more, 0.6% by mass or more, 0.8% by mass or more, 1.0% by mass or more, and may be 4.0% by mass or less, 3.8% by mass or less, 3.6% by mass or less, 3.4% by mass or less, 3.2% by mass or less, 3.0% by mass or less, 2.8% by mass or less, 2.6% by mass or less, 2.4% by mass or less, 2.2% by mass or less, or 2.0% by mass or less.

The (total) content of these polyorganic acids and polyorganic acid salts may be 2 parts by mass or more and 20 parts by mass or less relative to 100 parts by mass of pigment. It is preferable from the viewpoint of improving the dispersion stability of the pigment, particularly a titanium oxide pigment, that this (total) mass be 2 parts by mass or more, 3 parts by mass or more, 4 parts by mass or more, 5 parts by mass or more, 6 parts by mass or more, 7 parts by mass or more, or 8 parts by mass or more. It is preferable from the viewpoint of improving the viscosity range of the water-based liquid cosmetic and the viewpoint of inhibiting pigment sedimentation when stored in a pen-style applicator having a brush-shaped or pen-core-shaped application portion that this (total) mass be 20 parts by mass or less, 18 parts by mass or less, 15 parts by mass or less, 13 parts by mass or less, 12 parts by mass or less, or 11 parts by mass or less.

### <Emulsion particle>

The emulsion particle used in the present invention is an emulsion particle comprising an acrylates copolymer. Such an emulsion particle is also known as a film-forming polymer emulsion particle.

As such an emulsion particle, water-insoluble polymer emulsions, for example, styrene-alkyl acrylate copolymer emulsion, styrene-alkyl methacrylate copolymer, and copolymer emulsion consisting of acrylic acid, methacrylic acid, or a (C₁ to C₄ and C₈) alkyl ester component thereof, can be used.

As such an emulsion particle, emulsion particles of a composite polymer, such as a core-shell polymer emulsion consisting of a copolymer of an ethylenically unsaturated carboxylic acid monomer and a styrene-based monomer and another polymer and/or copolymer, can be used.

As the above styrene-alkyl acrylate copolymer emulsion, a (styrene/acrylates) copolymer can be used. As a commercially available product of this polymer, Yodosol GH41F (Nouryon Japan K.K.) and Daitosol 5000STY (Daito Kasai Kogyo Co., Ltd.) can be used.

Specific examples of emulsions of a core-shell polymer emulsion consisting of a copolymer of an ethylenically unsaturated carboxylic acid monomer and a styrene monomer and another polymer and/or copolymer include Emapoly CE-119N (Gifu Shellac Manufacturing Co., Ltd.), which is an emulsion of a core-shell copolymer of ethylhexyl acrylate/methacrylic acid copolymer [ammonium (acrylates/methylstyrene/styrene) copolymer].

Preferably, from the point of film formability and adhesion of drawn lines, those comprising an acrylates copolymer is preferable, and among these, the above core-shell polymer emulsion is even more preferable.

These emulsion particles, generally, have a resin component finely dispersed as solid content in an aqueous component at a concentration of 20 to 60% by mass. The content thereof, in terms of concentration based on solid content, may be 1% by mass or more and 30% by mass or less relative to the mass of the water-based liquid cosmetic. It is preferable from the viewpoint of improving adhesion and water-resistant adhesion that this content be 1% by mass or more, 2% by mass or more, 3% by mass or more, 4% by mass or more, or 5% by mass or more. It is preferable from the viewpoint of not excessively increasing the viscosity of the water-based liquid cosmetic and thereby improving applicability that this content be 30% by mass or less, 25% by mass or less, 20% by mass or less, 15% by mass or less, 12% by mass or less, 10% by mass or less, 9% by mass or less, 8% by mass or less, or 7% by mass or less.

### <Water-based solvent>

The water-based solvent used in the present invention is a solvent containing water as the main component, i.e., at a content of 50% by mass or more and 100% by mass or less relative to the mass of the water-based solvent, for example, 50% by mass or more, 60% by mass or more, 65% by mass or more, 70% by mass or more, 75% by mass or more, 80% by mass or more, or 82% by mass or more. The water-based solvent may be composed of water alone, or may further contain an organic solvent. When the water-based solvent contains an organic solvent, the content of water in the water-based solvent may be less than 100% by mass, 99% by mass or less, 95% by mass or less, 90% by mass or less, or 87% by mass or less relative to the mass of the water-based solvent. Due to the content of water above, satisfactory stability over time and dispersibility of the titanium oxide pigment can be obtained.

Particularly, the water-based solvent used in the present invention may contain water and at least one alcohol selected from the group consisting of lower alcohols having 10 or fewer carbon atoms and polyhydric alcohols having 10 or fewer carbon atoms.

As water, distilled water, ion-exchanged water, purified water, pure water, and ultrapure water can be used.

As the lower alcohols having 5 or fewer carbon atoms, specifically, at least one of methyl alcohol (methanol), ethyl alcohol (ethanol), n-propyl alcohol, isopropyl alcohol, n-butyl alcohol, sec-butyl alcohol, tert-butyl alcohol, isobutyl alcohol, pentyl alcohol, ethylene glycol, and propylene glycol can be used.

As the polyhydric alcohols having 10 or fewer carbon atoms, for example, glycerin, erythritol, threitol, arabinitol, xylitol, ribitol, maltitol, lactitol, mannitol, propylene glycol, 1,3-butylene glycol, 1,2-pentanediol, 1,2-hexanediol, 1,2-octanediol, ethylene glycol, diethylene glycol, dipropylene glycol, pentaerythritol, and ethylhexylglycerin can be used.

Among these, it is preferable from the point of safety and handleability that 1,3-butylene glycol be used.

The total content of the water-based solvent used may be 45% by mass or more and 85% by mass or less relative to the mass of the water-based liquid cosmetic. It is preferable from the viewpoint of demonstrating smooth applicability and moisturizing property and the viewpoint of exhibiting a slight preservative effect that this total content be 45% by mass or more, 50% by mass or more, 55% by mass or more, 60% by mass or more, or 63% by mass or more. It is preferable from the viewpoint of improving dispersion stability, particularly stability at low temperatures, that this total content be 85% by mass or less, 80% by mass or less, or 77% by mass or less.

### <Pigment other than titanium oxide pigment>

As pigments other than the titanium oxide pigment, inorganic pigments, such as inorganic coloring pigments, inorganic extender pigments, and inorganic pearlescent pigments, and organic pigments can be used. These pigments may be used independently, or may be used in combination.

As inorganic coloring pigments, for example, carbon black, iron oxide, titanium black, zinc oxide, talc, chromium oxide, cobalt oxide, titanated mica, bismuth oxychloride, ultramarine, and Prussian blue can be used.

As inorganic extender pigments, for example, talc, muscovite, phlogopite, lepidolite, biotite, synthetic mica, sericite, synthetic sericite, kaolin, silicon carbide, smectite, aluminum oxide, magnesium oxide, zirconium oxide, antimony oxide, diatomaceous earth, aluminum silicate, magnesium aluminum metasilicate, calcium silicate, barium silicate, magnesium silicate, calcium carbonate, magnesium carbonate, hydroxyapatite, boron nitride, and silicon dioxide can be used.

As inorganic pearlescent pigments, iron oxide-coated mica, bismuth oxychloride, fish scale flakes, and aluminum powder can be used.

As organic pigments, for example, Red No. 202, Red No. 220, Red No. 226, Red No. 228, Yellow No. 401, Yellow No. 205, Yellow No. 4 Al Lake, Yellow No. 203 Al Lake, and Red No. 104 Al Lake can be used.

Particularly, the above inorganic coloring pigments and organic pigments can be used as coloring materials. For example, when carbon black is used, by the combined use with the titanium oxide pigment above, a gray color tone can be obtained. Commercially available pigment mixtures comprising carbon black can also be used without particular limitation, as long as the pigment mixtures are used in liquid cosmetics.

The average particle size of these pigments before dispersion may be 100 nm or more and 20000 nm or less. This average particle size, for example, may be 20000 nm or less, 15000 nm or less, 10000 nm or less, 8000 nm or less, 5000 nm or less, 3000 nm or less, 2000 nm or less, 1800 nm or less, 1500 nm or less, 1300 nm or less, 1200 nm or less, 1100 nm or less, 1000 nm or less, 900 nm or less, or 800 nm or less, and may be 100 nm or more, 200 nm or more, 300 nm or more, 400 nm or more, 500 nm or more, or 600 nm or more.

The content of the pigments other than the titanium oxide pigment may be 0% by mass or more and 18% by mass or less relative to the mass of the water-based liquid cosmetic. This content may be 0% by mass or more or 1% by mass or more, and may be 18% by mass or less, 15% by mass or less, 12% by mass or less, 10% by mass or less, or 9% by mass or less.

### <Nonionic surfactant>

As nonionic surfactants, for example, those in which polyoxyethylene (POE) is ether-bonded to an organic acid, glycerin, or a higher alcohol can be used. Among these, polyoxyethylene (POE) alkyl ethers such as laureths (polyoxyethylene lauryl ethers), ceteths (polyoxyethylene cetyl ethers), steareths (polyoxyethylene stearyl ethers), and beheneths (polyoxyethylene behenyl ethers) are preferably used. These nonionic surfactants may be used independently, or may be used in combination.

Among these, it is preferable from the viewpoint of molecular structure and hydrophilicity that POE(20) cetyl ether (ceteth-20), POE(30) behenyl ether (beheneth-30), POE(20) stearyl ether, and POE(10) oleyl ether be used.

The content of the nonionic surfactant used may be 0.01% by mass or more and 1.00% by mass or less relative to the mass of the water-based liquid cosmetic, from the viewpoint of inhibiting excessive penetration into skin (so-called spidering) and the point of dispersion stability of the titanium oxide pigment. It is preferable that this content be 0.01% by mass or more, 0.03% by mass or more, 0.05% by mass or more, 0.07% by mass or more, or 0.09% by mass or more, and be 1.00% by mass or less, 0.90% by mass or less, 0.80% by mass or less, 0.70% by mass or less, 0.60% by mass or less, 0.50% by mass or less, 0.40% by mass or less, 0.35% by mass or less, or 0.30% by mass or less.

### <<Applicator>>

The applicator of the present invention is a pen-style applicator in which the above water-based liquid cosmetic is stored.

Particularly, the applicator of the present invention may comprise a front barrel including an application portion and a barrel tube portion including a reservoir portion. In this reservoir portion, the above water-based liquid cosmetic is stored (filled).

The applicator of the present invention needs only to be an applicator for cosmetics, and is not particularly limited. Particularly, specific aspects of the applicator to be described with reference to the drawings can be used as those of eyeliners or eyebrows. The applicator of the present invention may be eyeshadows and mascaras.

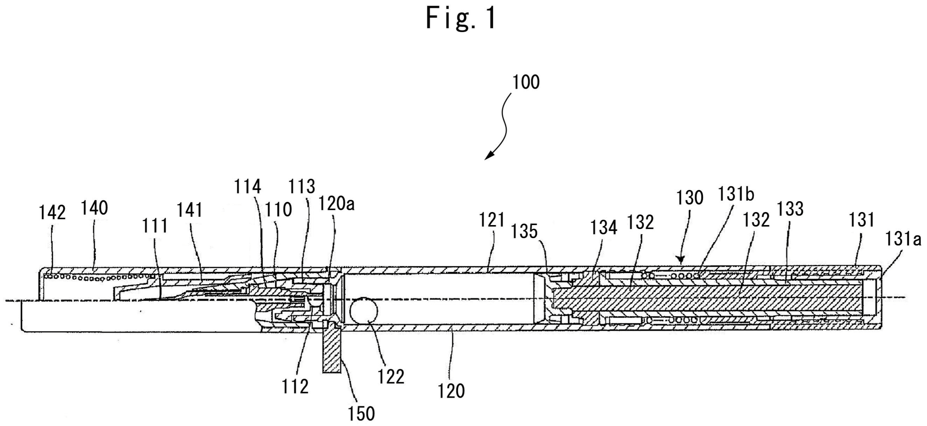

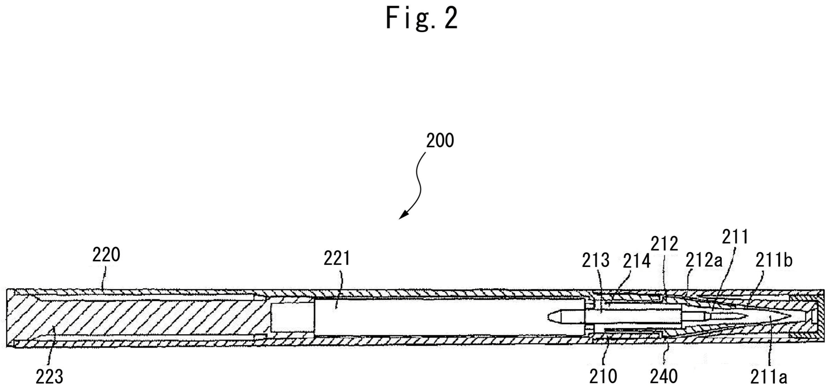

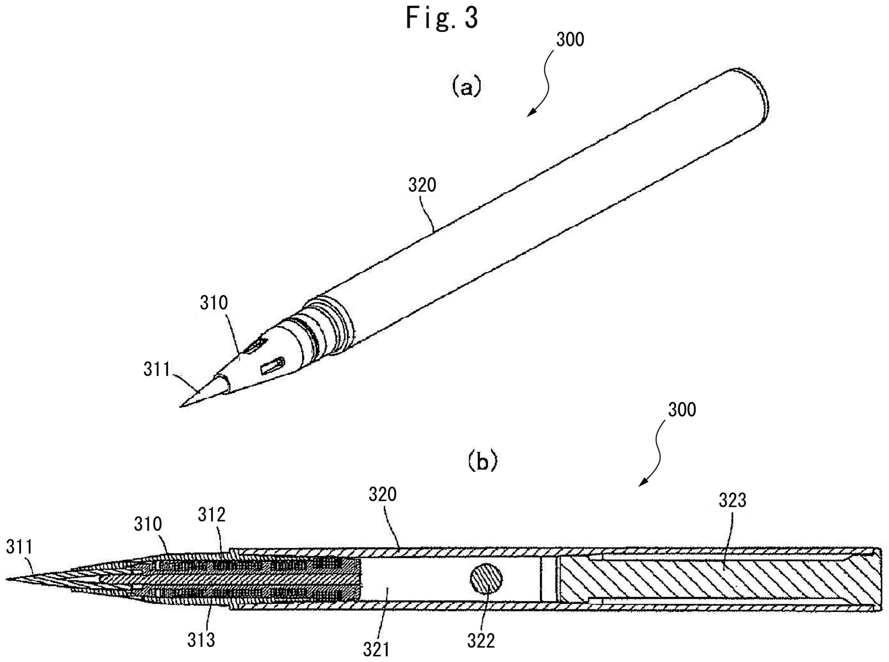

The applicator of the present invention, for example, may be an applicator of a rotary delivery type (FIG. 1), a knock delivery type (not illustrated), a cosmetic-integrated type (FIG. 2), or a collector type (FIG. 3).

Hereinafter, each configuration of the applicator of the present invention will be described.

### <Front barrel portion>

The front barrel portion includes an application portion. The front barrel portion may be formed so that a cap is detachable.

The front barrel portion assists in supplying the water-based liquid cosmetic from the barrel tube portion to the application portion, and thus may include a core. This core may be composed of a capillary material, such as a synthetic resin fiber bundle, a natural fiber bundle, or a resin porous body.

### (Application portion)

The application portion may be brush-shaped, pen-core-shaped, or in another application body form. Particularly, when the application portion is brush-shaped, the applicator of the present invention is useful for eyeliners and eyebrows.

When the application portion is in another application body form, the application portion, for example, may be composed of rubber, an elastomer, or a resilient closed-cell body.

When the application portion is brush-shaped, the application portion can be obtained, for example, by aligning (sharpening) a plurality of resin fiber bundles into a brush shape, so that the tip portion progressively narrows, and then integrating the rear end portion by heat fusion and expanding the diameter into a flange shape.

When the application portion is pen-core-shaped, the application portion may be composed of a fiber bundle, or may be composed of a porous body made of resin.

The fibers constituting the application portion may be synthetic fibers, or may be natural fibers. The material of the synthetic fibers, for example, may be polybutylene terephthalate (PBT). The thickness of the fibers may be 0.10 mm or more and 0.15 mm or less, and may be 0.10 mm or more or 0.11 mm or more and 0.15 mm or less or 0.14 mm or less.

### <Barrel tube portion>

The barrel tube portion includes a reservoir portion. In this reservoir portion, the above water-based liquid cosmetic is stored.

### (Reservoir portion)

The reservoir portion may have a hollow shape that can be filled with the water-based liquid cosmetic, or may be in the form of batting that can be impregnated with the water-based liquid cosmetic. When the reservoir portion has a hollow shape, the applicator is sometimes referred to as a "direct-liquid-type applicator". When the reservoir portion is in the form of batting, the applicator is sometimes referred to as a "batting-type applicator".

When the reservoir portion has a hollow shape, an agitating body may be stored in the reservoir portion. The agitating body may be in the form of a ball or rod. The agitating body may be made of metal, or may be made of resin.

### <Cap>

The cap is a member for covering the application portion when not in use. The cap may include an inner cap and a spring. The inner cap is a cup-shaped member that is disposed to be movable back and forth to improve airtightness of the application portion. The spring is a member for biasing this inner cap rearward.

### <Other configurations>

Regarding other optional configurations constituting the applicator of the present invention, the following descriptions of specific aspects can be referenced. These configurations may be resin-molded products.

Hereinafter, specific aspects of the applicator will be described with reference to the drawings. For optional configurations in the applicator of the present invention, the following descriptions can be appropriately referenced.

### <<Applicator of first aspect: rotary delivery type (FIG. 1)>>

The rotary delivery type applicator 100, as shown in FIG. 1, is an aspect in which the water-based liquid cosmetic of the present invention is discharged to a brush-shaped application portion 111 by a liquid pressing mechanism 130. This aspect can be used for a direct-liquid-type eyeliner. In front of the liquid pressing mechanism 130, a reservoir portion 121 in which the water-based liquid cosmetic is stored is present, and in front thereof, the application portion 111 is disposed.

The liquid pressing mechanism 130 delivers the water-based liquid cosmetic within the reservoir portion 121 for supplying to the application portion 111 by rotating a delivery member 131, arranged at the rear end portion of a barrel tube portion 120, in the circumferential direction relative to the barrel tube portion 120.

The liquid pressing mechanism 130 of this applicator 100 comprises a delivery member 131, a threaded rod 132, a drive tube 133, a threaded body 134, and a piston body 135. The delivery member 131 is rotatably fitted in the rear end of the barrel tube portion 120. The drive tube 133 can transmit a rotational force of the delivery member 131 by the user to the threaded rod 132. The threaded body 134 is fixed to the barrel tube portion 120 and threadedly engaged with the threaded rod 132. The threaded rod 132 has the piston body 135 rotatably engaged at the tip. The piston body slides within the reservoir portion 121 of the barrel tube portion 120. The rotation of the delivery member 131 is transmitted via the drive tube 133 to the threaded rod 132, and the threaded rod 132 and the piston body 135 are advanced via the female threads of a nut-like threaded body 134 by the rotation of the threaded rod 132, thereby discharging the water-based liquid cosmetic from within the reservoir portion 121 to the application portion 111.

The delivery member 131, as shown in FIG. 1, is exposed in a state of being rotatably fitted to the rear end portion of the barrel tube portion 120. A crown 131a is inserted into this rear end, thereby imparting the delivery member 131 an operable, closed cylindrical shape. The drive tube 133 is inserted within the delivery member 131, and is fixed in the rotational direction. The threaded body 134 is mounted within this drive tube 133, and the threaded body 134 is fixed in the rotational direction relative to the drive tube 133 and is capable of relative movement in the axial direction. A spring member 131b can bias the delivery member 131, acting as a rotary body, rearward.

"Fixed in the rotational direction" means that, when one member is mounted to another member, the one member is fixed so as to not rotate relative to the other member.

In this applicator, a sealing portion 113, a coupling member 114, a front barrel portion 110, and the application portion 111 are attached by inserting into the front end portion 120a of the barrel tube portion 120. The water-based liquid cosmetic is stored in the reservoir portion 121 of the barrel main body 120. The water-based liquid cosmetic delivered from the storage portion 121 is discharged to the application portion 111 through a flow path within the coupling member 114 and thus can be applied. After use, a cap 140 is mounted onto the front barrel portion 110, allowing the application portion 111 and the front barrel portion 110 to be covered.

As shown in FIG. 1, the barrel tube portion 120 may include an agitating ball 122 within the storage portion 121. According thereto, the water-based liquid cosmetic can be agitated by a reciprocating motion. The cap 140 may include an inner cap 141 provided on the inside thereof, and a spring 142 that can bias the inner cap 141 rearward.

When not in use (during distribution), in order to close the distribution path of the water-based liquid cosmetic toward the application portion 111, the applicator of the first aspect may include a stopper 150 and a sealing ball 112.

The stopper 150 has a ring-like portion, and when not in use, this ring-like portion is disposed between the rear end of the front barrel portion 110 and the front surface of a stepped section of the front end portion 120a of the barrel tube portion 120. This stopper 150 fixes the positions of the sealing portion 113, coupling member 114, front barrel portion 110, and application portion 111, enabling the distribution path of the water-based liquid cosmetic toward the application portion 111 to be closed.

This stopper 150 may have a portion of the ring-like portion cut away, and a tab on the side opposite the cut-away section may be integrally formed. When the tab is pulled, the ring-like portion expands in diameter from the cut-away section, allowing removal from between the rear end of the front barrel portion 110 and the front end portion 120a of the barrel main body 120.

The sealing ball 112, when not in use, is inserted into an inner diameter portion of the sealing portion 113, whereby the water-based liquid cosmetic can be prevented from flowing into the application portion 111 side.

During use, the user pulls off the stopper 150 from the barrel tube portion 120 and pushes the front barrel portion 110 to the rear end side, causing the rear end narrow portion of the coupling member 114 to abut against the sealing ball 112. The sealing ball 112 is then removed from the inner diameter portion of the sealing portion 113 and pushed into the reservoir portion 121. Accordingly, the water-based liquid cosmetic within the reservoir portion 121 flows from the inner diameter portion of the coupling member 114 into the liquid flow path of the application portion 111 and is thereby supplied through the flow path to the application portion 111, enabling application to a target area.

### <<Applicator of second aspect: cosmetic-integrated applicator type (FIG. 2)>>

The cosmetic-integrated applicator type applicator 200, as shown in FIG. 2, is an aspect having a structure for supplying the water-based liquid cosmetic in the inner portion of a barrel tube portion 220 to an application portion 211. This applicator 200 comprises the barrel tube portion 220, a front barrel portion 210, the application portion 211, and a retaining member 212. The barrel tube portion 220 stores the water-based liquid cosmetic by impregnation in a batting-type reservoir portion 221. The application portion 211 is provided in the front barrel portion 210, and can apply the water-based liquid cosmetic onto a target. The retaining member 212 is a member covering the outer periphery on the barrel tube portion 220 side (base portion side) of the application portion 211 except for the tip portion 211a of the application portion 211. This applicator 200 may have a cap 240 configured to be detachable from the tip portion 210, thereby allowing the application portion 211 and the retaining member 212 to be covered.

The batting-type reservoir portion 221 is housed within the barrel tube 220 from the center portion to the front barrel portion 210, and is sealed and supported by a tail plug 222 inserted into the rear end of the barrel tube 220.

A core 213 is disposed at an opening of the front barrel portion 210. This core may be formed from an open-cell foam. The tail end of this core 213 is fitted into the tip portion of the reservoir portion 221. The tip of this core 213 is inserted into the tail end portion of the application portion 211, thereby allowing the water-based liquid cosmetic stored in the reservoir portion 221 to be introduced to the application portion 211. The front barrel portion 210 has a slightly smaller diameter (diameter reduced by the thickness of the cap 240) than the barrel tube portion 220, thereby forming a step from the barrel tube portion 220. A core 213 is mounted within this front barrel portion 210 via a substantially tubular support body 214. The tubular rear end portion of the retaining member 212 is inserted between the outer periphery of the support body 214 and the inner periphery of the front barrel portion 210.

The tip portion of the retaining member 212 is positioned forward of the front barrel portion 210 to cover the outer peripheral surface 211b of the application portion 211. The outer peripheral surface 212a of the retaining member 212 progressively narrows into a conical side surface or a taper.

### <<Applicator of third aspect: collector applicator type (FIG. 3)>>

The collector applicator type applicator 300, as shown in FIG. 3(b), is an aspect in which a collector 312 is disposed at the front portion of a barrel tube portion 320 and a reservoir portion 321 is housed within the barrel tube portion 320. The collector 312 has a shape in which a plurality of leaf-like sheet portions are disposed in the axial direction so as to form comb-like shapes. As shown in FIG. 3(a), the front barrel portion 310 and the barrel tube portion 320 on the rear portion side of the front barrel portion 310 are fitted together.

The barrel tube 320 is formed into a pipe shape that has a communicating inner portion and is open at the front and rear. A tail plug 323 is fitted into the rear portion of the barrel tube portion 320, thereby closing the rear portion of the barrel tube portion 320. Accordingly, the barrel tube portion 320 interposed between the front end of the tail plug 323 and the rear end of the collector 312 forms the reservoir portion 321.

Within this reservoir portion 321, no impregnating body such as batting is disposed, the water-based liquid cosmetic is directly stored, and an agitating body (such as a ball) 322 for agitating the water-based liquid cosmetic is disposed.

The collector 312 is configured to be covered and retained by the front barrel portion 310 and the barrel tube portion 320.

A brush-shaped application portion 311 exhibiting a progressively narrowing taper protrudes from an opening at the front end portion of the front barrel portion 310. The front barrel portion 310 is formed to progressively narrow to exhibit a substantially conical side surface, and the tip angle of the front barrel portion 310 may be formed to have substantially the same angle as the tip angle of the application portion 311.

The front barrel portion 310 allows a cap to be detachably fitted therewith, thereby allowing the application portion 311 to be covered.

In the inner portion of the hollow, progressively narrowly formed front barrel portion 310, a bellows-shaped collector 312 is arranged in the rear of the application portion 311, and a core 313 is disposed so as to pass through the hollow portion of this collector 312.

The core 313 does not protrude from the rear end portion of the collector 312 into the reservoir portion 321 of the barrel tube portion 320 (refer to FIG. 3(b)). The rear end surface of the collector 312 and the rear end surface of the core 313 are substantially flush, whereby the rear end of the core 313 does not protrude into the reservoir portion 321. Accordingly, volume within the reservoir portion 321 can be ensured. When an agitating body 322 is provided within the reservoir portion 321, the agitating body 322 does not collide with the core 313 even when moving within the reservoir portion 321. Accordingly, deformation of the core 313 is inhibited, and as a result, sufficient penetration of application liquid is achieved.

### EXAMPLES

The present invention will be specifically described according to the Examples and Comparative Examples. However, the present invention is not limited thereto.

### <<Preparation of water-based liquid cosmetic>>

A titanium oxide pigment and a polyorganic acid or polyorganic acid salt were added to purified water in the amounts shown in Table 1 and dispersed using a disperser (Labostar, Ashizawa Finetech Ltd.). After confirming that the average particle size of the titanium oxide pigment had reached 300 nm, other components described in Table 1 were added and mixed, whereby a water-based liquid cosmetic was prepared.

Details of materials noted as *1 to *9 in Table 1 are as follows.
*1: CR-50, Ishihara Sangyo Kaisha, Ltd.
*2: TALOX ABL-412HP/R-516P, Titan Kogyo, Ltd.
*3: containing the CR-50, TALOX ABL-412HP above
*4: DK BLACK No. 2, Daito Kasei Kogyo Co., Ltd.
*5: (Ethylhexyl acrylate/methyl methacrylate) copolymer [ammonium (acrylates/methylstyrene/styrene) copolymer] (Emapoly-CE-119N, Gifu Shellac Manufacturing Co., Ltd., solid content of 47%)
*6: DERMACRYL AQF, Nouryon Japan K.K.
*7: AQUADEW SPA-30B, Ajinomoto Healthy Supply Co., Inc.
*8: JURYMER AC-10SL, Toagosei Co., Ltd.
*9: DDP-10, Nikko Chemicals Co., Ltd.

The pH of the prepared water-based liquid cosmetic was measured using a glass electrode pH meter under the condition of temperature of 25°C. The viscosity of the prepared water-based liquid cosmetic was measured using a viscometer (ELD viscometer, Toki Sangyo Co., Ltd.) under the conditions of temperature of 25°C and rotational speed of 50 rpm (shear rate of 192 [s⁻¹]).

### <<Evaluation>>

An applicator as shown in FIG. 1 was filled with the prepared water-based liquid cosmetic to prepare a direct-liquid-type eyeliner.

### <Color payoff property (immediately after preparation)>

The water-based liquid cosmetic was applied to skin, and the appearance of the drawn line was confirmed visually. The evaluation criteria were as follows.
A: Color payoff was very good.
B: Color payoff was good.
C: Color payoff was not good.
D: Color payoff was poor.

### <Color payoff property (after one week of storage)>

After the applicator was kept in storage for one week with the cap facing upward, the water-based liquid cosmetic was applied to skin, and the appearance thereof was confirmed visually.
A: Color payoff was very good.
B: Color payoff was good.
C: Color payoff was not good, such that transparency was observed.
D: Transparency was observed, and color payoff was poor.

### <Liquid outflow property>

The water-based liquid cosmetic was applied to skin, and the outflow condition of the drawn line was confirmed visually. The evaluation criteria were as follows.
A: There were no issues with flow rate, moistness was observed, and the application portion was also satisfactory.
B: Although a decrease in flow rate was observed, there were no issues with application, and there was no deficiency in the application portion.
C: There was a decrease in flow rate, and slight deficiency was observed in the application portion.

### <Cap-off non-drying property>

After leaving to stand for 10 minutes with the cap removed, the water-based liquid cosmetic was applied to skin, and the appearance of the drawn line was evaluated. The evaluation criteria were as follows.
A: There was no fading, and the application portion was satisfactory.
B: Some fading was observed, and slight deficiency was observed in the application portion.
C: There was fading, and large deficiency was observed in the application portion.
D: There was remarkable fading, and the application portion cannot be visually confirmed.

### <Adhesion>

The water-based liquid cosmetic was applied to skin, and the drawn line was rubbed with the pad of a finger to evaluate adhesion. The evaluation criteria were as follows.
A: There were no changes in the drawn line due to rubbing with the pad of a finger.
B: Slight changes in the drawn line were observed due to rubbing with the pad of a finger.
C: Significant changes in the drawn line were observed due to rubbing with the pad of a finger.

### <Water-resistant adhesion>

The water-based liquid cosmetic was applied to skin. Water was then run over the drawn line, and the drawn line was rubbed with the pad of a finger to evaluate water-resistant adhesion. The evaluation criteria were as follows.
A: There were no changes in the drawn line due to rubbing with the pad of a finger.
B: Slight changes in the drawn line were observed due to rubbing with the pad of a finger.
C: Significant changes in the drawn line were observed due to rubbing with the pad of a finger.

### <Sebum resistance>

The water-based liquid cosmetic was applied to skin. Artificial sebum was adhered to the drawn line, and the drawn line was rubbed with the pad of a finger to evaluate sebum resistance. The evaluation criteria were as follows.
A: No changes in the drawn line were observed due to rubbing with the pad of a finger.
B: Slight changes in the drawn line were observed due to rubbing with the pad of a finger.
C: Significant changes in the drawn line were observed due to rubbing with the pad of a finger.

Configurations and evaluation results of the Examples and Comparative Examples are shown in Tables 1 and 2.

### [Table 1]

**Table 1**

| | | Example 1 | Example 2 | Example 3 | Example 4 | Example 5 | Example 6 | Example 7 | Example 8 | Example 9 | Comparative Example 1 | Comparative Example 2 | Comparative Example 3 | Comparative Example 4 |
|---|---|---|---|---|---|---|---|---|---|---|---|---|---|---|
| Coloring material | Titanium oxide *¹ | 19 | 19 | 19 | 11.55 | 3.72 | 10.64 | 6.25 | 10.64 | 11.31 | 19 | 19 | 19 | 19 |
| | Iron oxide *2 | | | | 2.71 | 0.43 | 8.2 | 5.36 | 8.2 | 3.46 | | | | |
| | Aluminum hydroxide *3 | 1 | 1 | 1 | 0.61 | 0.2 | 0.56 | 0.33 | 0.56 | 0.54 | 1 | 1 | 1 | 1 |
| | Carbon black *4 | | | | 0.73 | 0.78 | | | | | | | | |
| | Red 226 | | | | | 2.49 | | | | | | | | |
| Emulsion resin | Core-shell acrylates copolymer *5 | 11.75 | 11.75 | 11.75 | 14.1 | 14.1 | 7.05 | 14.1 | 7.05 | 7.05 | 11.75 | 11.75 | 11.75 | 11.75 |
| | Acrylates copolymer *6 | | | | | | | | | | 3 | 5 | | |
| Polyorganic acid (salt) | Sodium polyaspartate *7 | 1.5 | 0.5 | | 1.66 | 0.43 | 1.85 | 0.65 | 1.85 | 1.7 | | | | |
| | Polyacrylic acid *8 | | | 3 | | | | | | | | | | |
| Nonionic surfactant | POE(20) cetyl ether | | | | 0.05 | 0.09 | 0.17 | 0.14 | 0.17 | 0.09 | | | | |
| | POE(30) behenyl ether | | | | 0.2 | 0.21 | | | | | | | | |
| Additive component | Citric acid | | | | 0.03 | | 0.03 | 0.03 | | | | | | |
| | AMP | | | 3 | | 0.04 | | | | | 0.2 | 0.33 | | |
| | Sodium dehydroacetate | | | | 0.01 | 0.01 | | | | | | | | |
| | Di(C12-15) pareth-10 phosphate *9 | | | | | 0.5 | | | | | | | 1 | 3 |
| | EDTA-2Na | | | | 0.11 | 0.11 | 0.04 | 0.02 | | | | | | |
| | Xanthan gum | | | | | 0.01 | | 0.02 | | | | | | |
| Preservative | Phenoxyethanol | 0.5 | 0.5 | 0.5 | 0.5 | 0.51 | 0.5 | 0.5 | 0.5 | 0.5 | 0.5 | 0.5 | 0.5 | 0.5 |
| | Methylparaben | 0.2 | 0.2 | 0.2 | 0.2 | 0.19 | 0.19 | 0.15 | 0.13 | 0.16 | 0.2 | 0.2 | 0.2 | 0.2 |
| | Ethylparaben | 0.11 | 0.11 | 0.11 | 0.13 | 0.13 | 0.11 | 0.1 | 0.09 | 0.08 | 0.11 | 0.11 | 0.11 | 0.11 |
| | 1,2-Hexanediol | 1 | 1 | 1 | 1 | 1 | | | | 1 | 1 | 1 | 1 | 1 |
| | Ethylhexylglycerin | | | | | | 0.18 | 0.17 | 0.18 | 0.13 | | | | |
| Water-based solvent | 1,3-Butylene glycol | 10 | 10 | 10 | 10.48 | 10.08 | 9.96 | 9.58 | 9.4 | 8.43 | 10 | 10 | 10 | 10 |
| | Water (purified water) | 54.94 | 55.94 | 50.44 | 55.93 | 64.97 | 60.52 | 62.6 | 61.23 | 65.55 | 53.24 | 51.11 | 55.44 | 56.44 |

### [Table 2]

**Table 2**

| | | Example 1 | Example 2 | Example 3 | Example 4 | Example 5 | Example 6 | Example 7 | Example 8 | Example 9 | Comparative Example 1 | Comparative Example 2 | Comparative Example 3 | Comparative Example 4 |
|---|---|---|---|---|---|---|---|---|---|---|---|---|---|---|
| Coloring material | Titanium oxide * 1 | 19 | 19 | 19 | 11.55 | 3.72 | 10.64 | 6.25 | 10.64 | 11.31 | 19 | 19 | 19 | 19 |
| | Other than titanium oxide | 1 | 1 | 1 | 4.05 | 3.9 | 8.76 | 5.69 | 8.76 | 4 | 1 | 1 | 1 | 1 |
| Emulsion resin | Core-shell acrylates copolymer *5 | 11.75 | 11.75 | 11.75 | 14.1 | 14.1 | 7.05 | 14.1 | 7.05 | 7.05 | 11.75 | 11.75 | 11.75 | 11.75 |
| | Acrylates copolymer *6 | | | | | | | | | | 3 | 5 | | |
| Polyorganic acid (salt) | Sodium polyaspartate *7 | 1.5 | 0.5 | | 1.66 | 0.43 | 1.85 | 0.65 | 1.85 | 1.7 | | | | |
| | Polyacrylic acid *8 | | | 3 | | | | | | | | | | |
| Nonionic surfactant | POE(20) cetyl ether | | | | 0.05 | 0.09 | 0.17 | 0.14 | 0.17 | 0.09 | | | | |
| | POE(30) behenyl ether | | | | 0.2 | 0.21 | | | | | | | | |
| Additive component | AMP | | | 3 | | 0.04 | | | | | 0.2 | 0.33 | | |
| | Di(C12-15) pareth-10 phosphate *9 | | | | | 0.5 | | | | | | | 1 | 3 |
| | Other additive component | 0 | 0 | 0 | 0.15 | 0.13 | 0.07 | 0.07 | 0 | 0 | 0 | 0 | 0 | 0 |
| Preservative | | 1.81 | 1.81 | 1.81 | 1.83 | 1.83 | 0.98 | 0.92 | 0.9 | 1.87 | 1.81 | 1.81 | 1.81 | 1.81 |
| Water-based solvent | | 64.94 | 65.94 | 60.44 | 66.41 | 75.05 | 70.48 | 72.18 | 70.63 | 73.98 | 63.24 | 61.11 | 65.44 | 66.44 |
| Mass of titanium oxide/mass of pigment component | | 0.95 | 0.95 | 0.95 | 0.74038 | 0.48819 | 0.54845 | 0.52345 | 0.54845 | 0.73873 | 0.95 | 0.95 | 0.95 | 0.95 |
| Mass of titanium oxide/mass of polyorganic acid salt | | 12.67 | 38.00 | 6.33 | 6.96 | 8.65 | 5.75 | 9.62 | 5.75 | 6.65 | - | - | - | - |
| Physical property | Viscosity (mPa·s) | 6.5 | 6.2 | 6.8 | 6.8 | 5 | 5.2 | 6.4 | 5 | 4.9 | 6.1 | 8.3 | 5.2 | 6.8 |
| | pH | 7.4 | 7.3 | 7.1 | 7.4 | 7.5 | 7.6 | 7.5 | 7.7 | 7.8 | 8.1 | 8 | 7.1 | 7.1 |
| Evaluation | Color payoff property | A | A | A | B | B | B | B | B | B | A | A | A | A |
| | Color payoff property (one week facing upward) | A | B | B | B | B | B | B | B | B | D | D | D | D |
| | Liquid outflow property | B | B | B | B | A | A | B | A | A | B | C | A | B |
| | Cap-off non-drying property | A | A | A | B | B | A | B | B | B | C | C | B | B |
| | Adhesion | B | B | B | A | A | B | A | B | B | B | B | B | B |
| | Sebum resistance | B | B | B | A | A | B | A | B | B | B | B | B | B |
| | Water-resistant adhesion | B | B | B | A | A | B | A | B | B | B | B | B | B |

It can be understood from Table 1 that in the cosmetics of Examples 1 to 9, color payoff property after being left facing upward for one week was excellent relative to the cosmetics of Comparative Examples 1 to 4. In addition, regarding color payoff property, liquid outflow property, cap-off non-drying property, adhesion, sebum resistance, and water-resistant adhesion, it can be understood that in the cosmetics of Examples 1 to 9, equivalent or better results with respect to the cosmetics of Comparative Examples 1 to 4 had been obtained.

### REFERENCE SIGNS LIST

100, 200, 300 applicator
110, 210, 310 front barrel portion
111, 211, 311 application portion
112 sealing ball
113 sealing portion
114 coupling member
120, 220, 320 barrel tube portion
120a front end portion of barrel tube portion
121, 221, 321 reservoir portion
122 agitating ball
130 liquid pressing mechanism
131 delivery member
131a crown
131b spring member
132 threaded rod
133 drive tube
134 threaded body
135 piston body
140, 240 cap
141 inner cap
142 spring
150 stopper
211a tip portion of application portion
211b outer peripheral surface of application portion
212 retaining member
212a outer peripheral surface of retaining member
213 core
214 support body
222 tail plug
312 collector
313 core
322 agitating body
323 tail plug

## Claims

1. A water-based liquid cosmetic stored in a pen-style applicator, wherein
a titanium oxide pigment, a polyorganic acid or polyorganic acid salt, an emulsion particle comprising an acrylates copolymer, and a water-based solvent are contained, and
a ratio of mass of the titanium oxide pigment relative to mass of the polyorganic acid or polyorganic acid salt is greater than 5.

2. The water-based liquid cosmetic according to Claim 1, wherein content of the titanium oxide pigment is 10% by mass or more relative to entirety of the water-based liquid cosmetic.

3. The water-based liquid cosmetic according to Claim 1 or 2, wherein the polyorganic acid or the polyorganic acid salt is at least one selected from a group consisting of polyaspartic acid, polylactic acid, polyphosphoric acid, and polyacrylic acid or salts thereof.

4. The water-based liquid cosmetic according to Claim 1 or 2, wherein the emulsion particle comprising the acrylates copolymer is a core-shell emulsion particle.

5. The water-based liquid cosmetic according to Claim 1 or 2, wherein the water-based solvent contains water and at least one alcohol selected from a group consisting of lower alcohols having 5 or fewer carbon atoms and polyhydric alcohols having 10 or fewer carbon atoms.

6. The water-based liquid cosmetic according to Claim 1 or 2, further containing a pigment other than the titanium oxide pigment.

7. The water-based liquid cosmetic according to Claim 6, wherein content of the titanium oxide pigment is 45% by mass or more relative to total amount of the pigment other than the titanium oxide pigment.

8. A pen-style applicator containing the water-based liquid cosmetic according to Claim 1 or 2.
